(19) Europäisches Patentamt
European Patent Office
Office européen des brevets

(11) **EP 1 985 271 A2**

(12) **EUROPEAN PATENT APPLICATION**

(43) Date of publication:
**29.10.2008 Bulletin 2008/44**

(51) Int Cl.:
*A61F 13/53* (2006.01)

(21) Application number: **08250837.5**

(22) Date of filing: **12.03.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **23.04.2007 US 789019**

(71) Applicant: **Tyco Healthcare Retail Services AG
8212 Neuhausen am Rheinfall (CH)**

(72) Inventors:
• **McVey, Dennis**
  **Lancaster, Pennsylvania 17601 (US)**
• **Driskell, Stacy J.**
  **Royersford, Pennsylvania 19468 (US)**

(74) Representative: **Elsy, David et al
Withers & Rogers LLP
Goldings House
2 Hays Lane
London
SE1 2HW (GB)**

(54) **Absorbent swim pant and core design for fluid movement**

(57)    A core for a swim pant garment includes a core having a ratio of total absorbent capacity (TAC) to total surface area (A) of the liquid receiving side of the core of less than about 2,500 g/m². The core may be formed of a plurality of hydrophobic fibers, including but not limited to polypropylene and/or cellulose acetate.

Fig. 1

EP 1 985 271 A2

**Description**

FIELD OF THE INVENTION

The invention relates generally to absorbent garments, and more particularly to an absorbent garment and core having a design for enhancing fluid movement.

BACKGROUND OF THE INVENTION

A number of commercially available diapers and undergarments are available for children and adults. Diapers and undergarments generally include a containment means to absorb liquids and capture solid wastes. Absorbent capacity is considered among the most important performance criteria of these products. Consumers desire products with high absorbent capacities to capture urine voids and absorb the liquid before the liquid can leak out of the product. Manufacturers are driven by consumers to select high absorbency materials, such as pulp fiber and superabsorbent polymers, to maximize the absorbent capacity of their products.

Many public swimming pools and recreational swimming areas require children to wear garments that prevent the release of fecal matter into the water. Fecal contamination of water in public pools can cause water-borne illnesses and pose a serious health risk to bathers who enter the water. Although diapers are capable of containing fecal matter, they have many drawbacks when used in pools or other play environments where water is prevalent. Diapers are primarily intended to remain dry, rather than submerged under water for a prolonged period of time. The outer material used on diapers can tear, and a tear at the absorbent core can release pulp fibers and superabsorbent polymer into the pool. Absorbent materials released from diapers are known to clog pool filters. Therefore, some facilities prohibit diapers in their pools.

The highly absorbent materials in diapers also create problems for children wearing diapers in pools. The highly absorbent core materials in diapers are purposefully designed to absorb a substantial amount of liquid. Pool water that is absorbed by the core material does not readily drain out of the core. The intake of liquid will cause the diaper to expand to several times its dry volume, while undergoing a significant increase in weight. Expansion of the diaper, often referred to as "swelling," can alter the fit of the diaper and impede the child's ability to move in or out of water. Once the child exits the water, the diaper may sag significantly under the added weight of water. Because the water does not readily drain from the absorbent core, the diaper will remain waterlogged and wet against the child's skin, creating the potential for rashes and discomfort. Under these conditions, the waterlogged diaper must be removed, even if the child will soon return to the water.

To address the problem of swelling, product manufacturers have introduced variations of diapers called "swim diapers" that are intended to be more suited for use in swimming pools. Like conventional diapers, swim diapers have cores designed to absorb significant amounts of liquid. Liquid that is absorbed by the core material does not readily move out of the core, and the core therefore remains wet against the skin for a prolonged period. This can cause rashes and discomfort.

In view of the foregoing drawbacks, existing diaper products for young children are less than desirable for use in swimming pools and recreational areas where the diaper can be exposed to water. Therefore, there remains a need for improved swim pant products.

SUMMARY OF THE INVENTION

The drawbacks of using diapers and other prior art garments for swimming are avoided to a large extent by a swim pant having a core for enhanced fluid movement. In a first exemplary embodiment, a core has a ratio of total absorbent capacity (TAC) to total surface area (A) of the liquid receiving side of less than about 2,500 $g/m^2$. The ratio of TAC/A may be from about 1,600 to about 2,500 $g/m^2$. Alternatively, the ratio of TAC/A may be from about 1,700 to about 2,000 $g/m^2$. Furthermore, the ratio of TAC/A maybe from about 1,800 to about 1,900 $g/m^2$.

The core may be formed of a plurality of hydrophobic fibers. The plurality of hydrophobic fibers may include non-woven polypropylene fiber. Alternatively, the plurality of hydrophobic fibers may include cellulose acetate fiber. The total surface area of the core may be from about 300 to about 450 $cm^2$. Moreover, the core may have a total absorbent capacity (TAC) of from about 40 to about 80 ml $H_2O$ or from about 40 to 80 g $H_2O$, for example from about 40 to about 60 g $H_2O$ or from about 40 to about 60 ml $H_2O$.

A core according to a second exemplary embodiment has a total absorbent capacity (TAC) and a liquid receiving side having a total surface area (A) in which the TAC is from about 40 to about 80 ml $H_2O$ or from about 40 to about 80 g $H_2O$. The core may have a ratio of total absorbent capacity (TAC) to total surface area (A) of the liquid receiving side of less than about 2,500 $g/m^2$. Alternatively, the core may have a ratio of TAC/A of from about 1,600 to about 2,500 $g/m^2$, from about 1,700 to about 2,000 $g/m^2$, or from about 1,800 to about 1,900 $g/m^2$. The core may include a plurality of hydrophobic fibers. The plurality of hydrophobic fibers may include non-woven polypropylene fiber. Alternatively, the plurality of hydrophobic fibers may include cellulose acetate fiber. The total surface area of the core may be from about 300 to about 450 cm$^2$. In addition, the core may have a TAC of from about 40 to about 60 ml $H_2O$, or from about 40 to about 50 ml $H_2O$.

A swim pant according to a third exemplary embodiment is a swim pant having enhanced fluid movement comprising:

a top sheet;

a back sheet; and

a core interposed between the top sheet and the back sheet, the core having a total absorbent capacity (TAC) and a liquid receiving side having a total surface area (A), wherein the ratio of TAC/A is less than about 2,500 g/m$^2$.

The core of the swim pant may be according to the first exemplary embodiment.
A swim pant according to a fourth exemplary embodiment is a swim pant having enhanced fluid movement, the swim pant comprising:

a top sheet;

a back sheet; and

a core interposed between the top sheet and the back sheet, the core having a total absorbent capacity (TAC) and a liquid receiving side having a total surface area (A), the core comprising a plurality of hydrophobic fibers and having a TAC of from about 40 to about 80 g $H_2O$.

The core of the swim pant may be according to the second exemplary embodiment.
A fifth exemplary embodiment is a method of forming a swim pant having enhanced fluid movement comprising the steps of:

(a) forming a core having a total absorbent capacity (TAC) and a liquid receiving side having a total surface area (A), wherein the ratio of TAC/A is less than about 2,500 g/m$^2$; and

(b) interposing the core between a top sheet and a back sheet.

The core may be according to the first exemplary embodiment.
A sixth exemplary embodiment is a method of forming a swim pant having enhanced fluid movement comprising the steps of:

(a) forming a core having a total absorbent capacity (TAC) and a liquid receiving side having a total surface area (A), the core comprising a plurality of hydrophobic fibers and having a TAC of from from about 40 to about 80 g $H_2O$; and

(b) interposing the core between a top sheet and a back sheet.

The core of the swim patent may be according to the second exemplary embodiment.

BRIEF DESCRIPTION OF THE DRAWINGS

The foregoing summary and the following description will be more clearly understood in conjunction with the drawing figures, in which:

Figure 1 is a perspective view of a swim pant in accordance with an exemplary embodiment of the present invention, where the swim pant is shown in an unopened condition;

Figure 2 is a plan view of the swim pant of Fig. 1, where the swim pant is shown in an opened condition;

Figure 3 is an enlarged cross-sectional view of the swim pant of Figs. 1 and 2, taken through line 3-3 of Fig. 2; and

Figure 4 is an enlarged plan view of a core component of the swim pant of Fig. 1.

DETAILED DESCRIPTION OF THE INVENTION

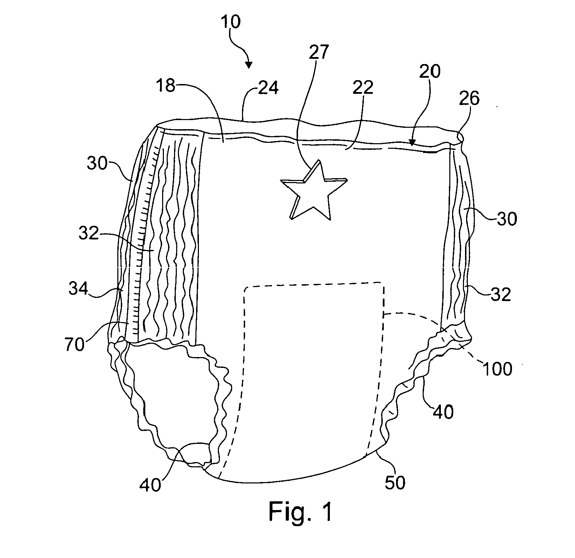

Referring now to Figure 1, a swim pant 10 in accordance with one embodiment of the invention is shown. Swim pant 10 has a body configuration that can be pulled up and over the legs of the wearer and secured around the wearer's waist. A waist portion 20 secures the swim pant 10 about the wearer's waist or abdominal area. A pair of side panels 30 extend from the waist portion. Side panels 30 stretch around the waist and thighs of the wearer and generally conform to the wearer's body shape. Each side panel 30 adjoins a leg opening 40 adapted to receive and surround one of the wearer's legs. The leg openings 40 are surrounded by cuffs 60 that fit snugly around the wearer's legs. A crotch portion 50 separates leg openings 40. A liquid retaining core 100 extends through the interior of the swim pant within the crotch portion 50.

Swim pant 10 is generally shown as it would appear in a ready-to-use condition. Waist portion 20 includes a front waist portion 22 and a rear waist portion 24. Similarly, side panels 30 each include a front side panel portion 32 and a rear side panel portion 34. In the ready-to-use condition, the front waist portion 22 adjoins the rear waist portion 24, while the front side panel portions 32 adjoin the rear side panel portions 34. Front and rear waist portions 22 and 24 collectively form a waist opening 26. To place swim pant 10 on the wearer, the wearer's feet are inserted through the waist opening, through the body of the swim pant and out each of the leg openings 40. Swim pant is then advanced up the wearer's legs to the wearer's crotch region, with the waist opening 26 secured around the wearer's waist and/or abdominal area by waist portion 20.

Each side panel 30 includes a tear seam or section 70. The tear sections 70 are formed of material that can be torn or split by hand to separate the front and rear side panel portions 32 and 34 from one another. In this configuration, tear sections 70 can be split to remove swim pant 10 from the wearer without sliding the swim pant down the wearer's legs. In the preferred embodiment, front and rear side panel portions 32 and 34 are refastenable after they are torn or split apart. For example, tear seams 70 may include one or more areas containing adhesive, hook and loop fasteners, or other refastenable connectors along or adjacent to the tear seams 70. This feature may be desirable where the swim pant will be worn and removed multiple times.

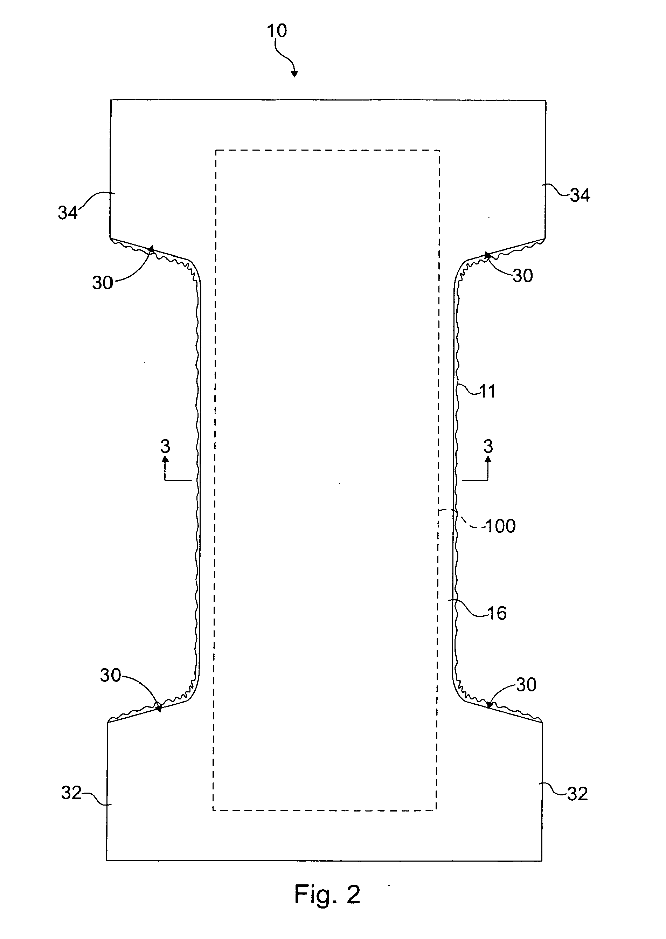

Referring now to Figure 2, swim pant 10 is shown in an opened condition after the side panels 30 are split apart at the tear portions 70. Swim pant 10 has an hourglass-shaped body 11 in the opened condition. The body 11 includes a top sheet 16 that contacts the wearer when swim pant 10 is worn. Top sheet 16 may be formed of a nonwoven material. Preferably, top sheet 16 includes one or more coatings of aloe, vitamin E or other coating materials that promote skin wellness. Swim pant also includes a back sheet 18 that faces away from the wearer when worn, forming the exterior of the garment as shown in Fig. 1. Back sheet 18 may be formed of a hydrophobic nonwoven material. A decorative appliqué may be applied to back sheet 18, such as the appliqué design 27.

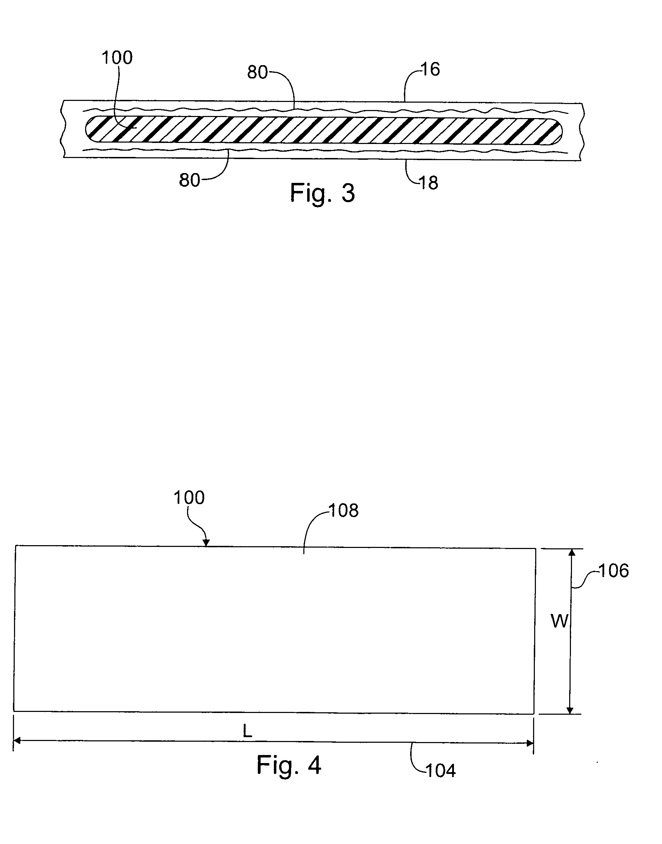

Referring now to Fig. 3, core 100 extends between top sheet 16 and back sheet 18. Core 100 is surrounded on each side by a tissue layer 80 formed of a cellulose material. Referring to Fig. 4, core 100 has a liquid receiving side 108 having a length "L" 104, a width "W" 106 and a total surface area 110. Liquid receiving side 108 is arranged to face inwardly toward the wearer when swim pant 10 is worn.

One of the advantages of swim pant 10, unlike diapers and other swim garments, is the swim pant's ability to be absorbent when needed, and dry quickly when needed. Swim pant 10 addresses the need for absorbing voids without providing the undesired water retention encountered in known swim garments. Under dry conditions, swim pant 10 acts like a diaper, with a core 100 having enough capacity to capture a void. Under wet conditions, such as when the wearer exits a swimming pool after being submerged, the core allows pool water to be released so that the core can dry. This limits the potential for skin rashes and other adverse effects.

The drying rate of a particular swim pant is dependent on several factors. Dry times are fairly subjective, and can vary based on humidity, temperature and other variables in the environment of the test location. Swim pants in accordance with the invention were submerged in water and allowed to sit at room temperature and pressure. The liquid receiving side of the cores were dry to the touch in less than 24 hours. Diaper cores that were subjected to the same testing remained water-logged and were wet to the touch after the same 24 hour period.

Drying rate and liquid retention are competing properties in a swim garment. As more and more liquid is retained in the swim garment, the longer the expected dry time will be. It has been discovered that core drying rate and liquid retention can be appropriately balanced to meet the needs of young swimmers who rely on a diaper-type product to contain bowel movements. It has further been discovered that the balance between core drying rate and liquid retention can be reflected by an index reflecting the ratio of total absorbent capacity of the core to surface area of the core. This index, referred to as the "drying index," is summarized below as:

$$I_{dr} = TAC/A$$

where $I_{dr}$ = the drying index (g/m$^2$);

TAC = total absorbent capacity (g H$_2$O); and

A = total surface area of liquid receiving side of core (m$^2$).

It has been found that a drying index $I_{dr}$ of from about 1,600 to about 2,500 g/m$^2$ can provide both a fast drying rate and a sufficient absorbent capacity to meet the needs of child swimmers. Swim pants were tested in three different sizes, small "S", medium "M", and large "L." All three sample swim pants were found to exhibit fast drying rates compared to diaper products. In addition, the three samples exhibited absorbent capacities that were sufficiently large to contain at least one void. Information on each of the sample swim pants is summarized in the following table.

TABLE 1

|  | TAC (g H$_2$O) | Core L (mm) | Core w (mm) | $I_{dr}$ (g/m$^2$) |
|---|---|---|---|---|
| Swim Pant "S" | 70 | 365 | 100 | 1918 |
| Swim Pant "M" | 71 | 385 | 100 | 1844 |
| Swim Pant "L" | 72 | 435 | 100 | 1655 |

The total absorbent capacities of all three samples were roughly equal, in the range of 70-72 g H$_2$O. This capacity reflects or exceeds a conservative estimate of what is needed to retain most voids under dry conditions, assuming an average void of approximately 40 ml.

The length "L" of the core's liquid receiving side varies for each of the samples, with the length being a minimum with the small swim pant, and a maximum with the large swim pant. The width "W" of the core's liquid receiving side is the same in each sample. The total surface area of the liquid receiving sides of the samples varies from about 365 cm$^2$ to about 435 cm$^2$. The calculated drying index, $I_{dr}$ is largest in the small swim pant and smallest in the large swim pant.

Swim pants with values for TAC, L, W and $I_{dr}$ that are higher or lower than those in the above three samples may still demonstrate satisfactory performance and therefore be used in accordance with the invention. For example, total surface area can be from about 300 cm$^2$ to about 450 cm$^2$, or smaller or larger. Nonetheless, performance of the swim pant may diminish if the drying index is too large or too small. For example, a low drying index associated with a very low absorbent capacity can provide a fast dry time but not provide sufficient capacity to hold a void.

The TAC value for the core is preferably from about 40 to about 80 ml H$_2$O, more preferably from about 40 to about 60 ml H$_2$O, and still more preferably from about 40 to about 50 ml H$_2$O. The $I_{dr}$ is preferably from about 1,600 to about 2,500 g/m$^2$, more preferably from about 1,700 to about 2,000 g/m$^2$, and still more preferably from about 1,800 to about 1,900 g/m$^2$.

In a preferred embodiment, the core is formed of a hydrophobic fiber matrix. The selection of a hydrophobic fiber matrix in combination with a core design having a drying index $I_{dr}$ of from about 1,600 to about 2,500 g/m$^2$ provides both a fast drying rate and a sufficient liquid retention capacity. Hydrophobic fiber materials, such as polypropylene or cellulose acetate, repel water so that water is naturally released from the matrix, and resists soaking into the fibers. Liquid retention is attributed in part to some liquid being trapped between fibers. Water can move or flow out of the core by air drying, or by being forced out from pressure on the core.

Although a rectangular core is shown in the Figures, the core can have any shape and geometry that provides the desired drying rate and liquid retention properties described above. For example, the core may have a square shape, circular shape, elliptical or oval shape, or any polygonal shape. The core may also have a variety of different thicknesses, although thinner cores are preferred. Moreover, the core may be constructed using a variety of known techniques, including those used in making cores for conventional diaper cores. U.S. Patent No. 6,455,753 to Glaug et al. ("the '753 Patent"), the contents of which are incorporated by reference herein, describes a number of materials and techniques for constructing absorbent articles and cores for use in absorbent articles. The '753 Patent discusses ways to improve fluid acquisition, and uses superabsorbent polymers and other materials. High absorbency materials, such as superabsorbent polymers, are omitted from an exemplary embodiment of the swim pant illustrated in Fig. 1 to provide the desired balance between drying rate and liquid retention. Such high absorbency materials are, however, optionally included.

**Claims**

1. A core for enhanced fluid movement in a swim pant, the core having a total absorbent capacity (TAC) and a liquid receiving side having a total surface area (A), wherein the ratio of TAC/A is less than about 2,500 $g/m^2$.

2. The core of claim 1, wherein the ratio of TAC/A is from about 1,600 to about 2,500 $g/m^2$.

3. The core of claim 1 or 2, wherein the ratio of TAC/A is from about 1,700 to about 2,000 $g/m^2$.

4. The core of any one of claims 1 to 3, wherein the ratio of TAC/A is from about 1,800 to about 1,900 $g/m^2$.

5. The core of any one of claims 1 to 4, wherein the core is formed of a plurality of hydrophobic fibers.

6. The core of claim 5, wherein the plurality of hydrophobic fibers comprises non-woven polypropylene fiber.

7. The core of claim 5 or 6, wherein the plurality of hydrophobic fibers comprises cellulose acetate fiber.

8. The core of any one of claims 1 to 7, wherein the total surface area (A) of the core is from about 300 to about 450 $cm^2$.

9. The core of any one of claims 1 to 8, wherein the core has a TAC of from about 40 to about 80 g $H_2O$.

10. The core of any one of claims 1 to 9, wherein the core has a TAC of from about 40 to about 60 g $H_2O$.

11. A core for enhanced fluid movement in a swim pant, the core having a total absorbent capacity (TAC) and a liquid receiving side having a total surface area (A), the core comprising a plurality of hydrophobic fibers and having a TAC of from about 40 to about 80 g $H_2O$.

12. The core of claim 11, wherein the ratio of TAC/A is less than about 2,500 $g/m^2$.

13. The core of claim 11 or 12, wherein the ratio of TAC/A is from about 1,600 to about 2,500 $g/m^2$.

14. The core of any one of claims 11 to 13, wherein the ratio of TAC/A is from about 1,700 to about 2,000 $g/m^2$.

15. The core of any one of claims 11 to 14, wherein the ratio of TAC/A is from about 1,800 to about 1,900 $g/m^2$.

16. The core of any one of claims 11 to 15, wherein the plurality of hydrophobic fibers comprises non-woven polypropylene fiber.

17. The core of any one of claims 11 to 16, wherein the plurality of hydrophobic fibers comprises cellulose acetate fiber.

18. The core of any one of claims 11 to 17, wherein the total surface area of the core is from about 300 to about 450 $cm^2$.

19. The core of any one of claims 11 to 18, wherein the core has a TAC of from about 40 to about 60 g $H_2O$.

20. The core of any one of claims 11 to 19, wherein the core has a TAC of from about 40 to about 50 g $H_2O$.

21. A swim pant having enhanced fluid movement, the swim pant comprising:

    a top sheet;
    a back sheet; and
    a core interposed between the top sheet and the back sheet, wherein the core is according to any one of claims 1 to 10.

22. A swim pant having enhanced fluid movement, the swim pant comprising:

    a top sheet;
    a back sheet; and
    a core interposed between the top sheet and the back sheet, wherein the core is according to any one of claims 11 to 20.

23. A method of forming a swim pant having enhanced fluid movement comprising the steps of:

(a) forming a core according to any one of claims 1 to 10; and
(b) interposing the core between a top sheet and a back sheet.

24. A method of forming a swim pant having enhanced fluid movement comprising the steps of:

(a) forming a core according to any one of claims 11 to 20; and
(b) interposing the core between a top sheet and a back sheet.

Fig. 1

Fig. 2

Fig. 3

Fig. 4

**EP 1 985 271 A2**

**REFERENCES CITED IN THE DESCRIPTION**

**Patent documents cited in the description**

- US 6455753 B, Glaug